# EUROPEAN PATENT SPECIFICATION

(11) **EP 1 578 390 B1**
(45) Date of publication and mention of the grant of the patent: **28.02.2007**
(21) Application number: 03789259.3
(22) Date of filing: 10.12.2003
(51) Int. Cl.: A61K 8/44, A61K 8/46, A61K 8/49, A61Q 5/02

(54) **HAIR TREATMENT COMPOSITIONS CONTAINING CLIMBAZOLE**
HAARBEHANDLUNGSMITTEL ENTHALTEND CLIMBAZOLE
COMPOSITIONS DE TRAITEMENT CAPILLAIRE CONTENANT DU CLIMBAZOLE

(30) Priority: 23.12.2002 GB 0229733
(43) Date of publication of application: 28.09.2005
(73) Proprietor: UNILEVER PLC, London EC4P 4BQ (GB); UNILEVER N.V., 3013 AL Rotterdam (NL)
(72) Inventor: BAILEY, Peter, L., Unilever R & D Port Sunlight, Wirral, Merseyside CH63 3JW (GB); CHANG, Wanlin, Unilever Thai Holdings Ltd, Ladyao, Jatujak, 10900 Bangkok (TH); WILLIAMS, Alun Peter, Unilever R & D Port Sunlight, Wirral, Merseyside CH63 3JW (GB)
(74) Representative: Tansley, Sally Elizabeth
(86) International application number: PCT/EP2003/014180
(87) International publication number: WO 2004/056329

(56) References cited:
- EP-A- 0 338 850
- EP-A- 0 799 612
- WO-A-03/088940
- US-B1- 6 383 996

## Description

### FIELD OF THE INVENTION

This invention relates to hair treatment compositions and to methods of treating hair using the compositions.

### BACKGROUND AND PRIOR ART

Shampoo compositions generally contain a surfactant. The compositions may also contain an active agent such as an anti-dandruff agent. In order for the active agent to have improved effectiveness, it is necessary for the active agent to be deposited from the composition onto the hair and/or the scalp.

Some shampoo compositions are perceived by the users of the compositions to be harsh on the hair. There remains a need for compositions that have greater mildness.

There is also a need for shampoo compositions that have better deposition onto the hair and/or the scalp.

EP-A-0569028 describes a clear mild shampoo comprising a polyglyceryl ester as viscosity builder. In Example 1, the composition contains 6% sodium lauryl sulfate, 16% cocamidopropyl betaine and 2% each of sodium cocamphoacetate and disodium cocoamphodiacetate. There is no mention of anti-dandruff agents. A similar disclosure is found in US 5,478,490.

US 5,874,073 and US 6,297,203 disclose styling shampoo compositions comprising a hair styling polymer and other components. In the examples, lauramphoacetate and cocamidopropyl betaine are used as alternatives.

US 5,962,395 discloses liquid cleansing compositions comprising an anionic surfactant and an amphoteric and/or zwitterionic surfactant.

The present invention is based on the surprising finding that certain combinations of surfactants provide hair treatment compositions that are perceived by the user as being mild. The same combinations of surfactants unexpectedly also allow increased deposition of active agents.

### DESCRIPTION OF THE INVENTION

In a first aspect, the present invention provides a hair treatment composition comprising:
an anionic surfactant;
an amphoteric or zwitterionic surfactant;
an alkyl amphoalkanoate surfactant; and
an anti-dandruff agent that is in solution in the composition wherein the anti-dandruff agent is climbazole.

### DETAILED DESCRIPTION OF THE INVENTION

### Anionic surfactant

An anionic surfactant is one component of the compositions of the invention. Examples of suitable anionic surfactants which can be used as the anionic surfactant in compositions of the invention include cleansing surfactants , such as the alkyl sulphates, alkyl ether sulphates, alkaryl sulphonates, alkanoyl isethionates, alkyl succinates, alkyl sulphosuccinates, N-alkyl sarcosinates, alkyl phosphates, alkyl ether phosphates, alkyl ether carboxylates, and alphaolefin sulphonates, especially their sodium, magnesium, ammonium and mono-, di- and triethanolamine salts. The alkyl and acyl groups generally contain from 8 to 18 carbon atoms and may be unsaturated. The alkyl ether sulphates, alkyl ether phosphates and alkyl ether carboxylates may contain from 1 to 10 ethylene oxide or propylene oxide units per molecule.

Typical anionic cleansing surfactants for use as the anionic surfactant component in compositions of the invention include sodium oleyl succinate, ammonium lauryl sulphosuccinate, ammonium lauryl sulphate, sodium dodecylbenzene sulphonate, triethanolamine dodecylbenzene sulphonate, sodium cocoyl isethionate, sodium lauryl isethionate and sodium N-lauryl sarcosinate. The most preferred anionic surfactants are sodium lauryl sulphate, sodium lauryl ethersulphate(n)EO, (where n ranges from 1 to 3), ammonium lauryl sulphate and ammonium lauryl ether sulphate(n)EO, (where n ranges from 1 to 3).

Mixtures of any of the foregoing anionic surfactants may also be suitable for use as the anionic surfactant.

The anionic surfactant (including all anionic surfactants in a mixture of anionic surfactants) is preferablypresent in the compositions of the invention in an amount of from 5 to 25% by weight, more preferably from 10 to 20 % by weight, even more preferably from 11% to 16% by weight, most preferably from 13 to 15% by weight, of the composition.

### Amphoteric or zwitterionic surfactant

Another component of the compositions of the invention is an amphoteric or zwitterionic surfactant

Examples of amphoteric and zwitterionic surfactants include alkyl amine oxides, alkyl betaines, alkyl amidopropyl betaines, alkyl sulphobetaines (sultaines), alkyl glycinates, alkyl carboxyglycinates, alkyl amidopropyl hydroxysultaines, acyl taurates and acyl glutamates, wherein the alkyl and acyl groups have from 8 to 19 carbon atoms. Preferred amphoteric and zwitterionic surfactants for use in shampoos of the invention include lauryl amine oxide, cocodimethyl sulphopropyl betaine and preferably lauryl betaine and cocamidopropyl betaine. Cocamidopropyl betaine is especially preferred.

Mixtures of any of the foregoing amphoteric or zwitterionic surfactants may also be suitable for use as the amphoteric or zwitterionic surfactant.

The amphoteric or zwitterionic surfactant (including all amphoteric or zwitterionic surfactants in a mixture of amphoteric or zwitterionic surfactants) is preferably present in the compositions of the invention in an amount of from 0.1 to 10% by weight, more preferably from 0.5 to 5 % by weight, even more preferably from 1% to 4% by weight, most preferably from 1 to 3% by weight, of the composition.

### Alkyl amphoalkanoate surfactant

Compositions of the invention also comprise an alkyl amphoalkanoate which is considered herein separately from, and is chemically different from, the amphoteric or zwitterionic surfactant. Preferred are( C₆ to C₃₀ alkyl)ampho (C₁ to C₆ alkanoates), more preferably (C₆ to C₁₈ alkyl)ampho (C₁ to C₆ alkanoates), even more preferably (C₆ to C₁₈ alkyl)ampho (C₁ to C₂ alkanoates), most preferably (C₆ to C₁₈ alkyl)amphoacetates). Examples of the most preferred (C₆ to C₃₀ alkyl)ampho(C₁ to C₆ alkanoates) are cocoamphoacetates and laurylamphoacetates, with the sodium salts of these compounds being even more preferred, such as sodium cocoamphoacetate.

Alkyl amphoalkanoates such as alkyl amphoacetates are defined in the industry by the Cosmetic, Toiletry, and Fragrance Association, (CTFA).

Mixtures of any of the foregoing alkyl amphoalkanoate surfactants may also be suitable for use as the alkyl amphoalkanoate in the compositions of the invention

The amphoalkanoate surfactant (including all amphoalkanoate surfactants in a mixture of amphoalkanoate surfactants) is preferably present in the compositions of the invention in an amount of from 0.1 to 10% by weight, more preferably from 0.5 to 5 % by weight, even more preferably from 1% to 4% by weight, most preferably from 1 to 3% by weight, of the composition.

### Particularly preferred compositions

Particularly preferred compositions of the invention comprise a surfactant composition comprising: sodium lauryl sulphate or sodium lauryl ether sulphate(n)EO, (where n ranges from 1 to 3) in an amount of from 10 to 20 % by weight of the composition; cocamidopropylbetaine in an amount of from 0.5 to 5% by weight of the composition; and sodium cocoamphoacetate or sodium lauryl cocoamphoacetate in an amount of from 0.5 to 5% by weight of the composition.

### Anti-dandruff agent

Compositions of the invention comprise an anti-dandruff agent. Anti-dandruff agents include, for example, those compounds included in shampoo compositions for the purpose of treating or preventing dandruff, such as octopirox, climbazole and ketoconazole.

The anti-dandruff agent is in solution in the composition. The anti-dandruff agent is therefore preferably soluble in the composition of the invention at 25 degrees C at the level of anti-dandruff agent that is employed in the composition. The anti-dandruff agent is climbazole (1-imidazolyl-1-(4-chlorophenoxy)-3,3-dimethylbutan-2-one).

Preferably, the anti-dandruff agent is present in the composition in an amount of from 0.1 to 5% by weight, more preferably from 0.1 to 2% by weight.

It has surprisingly been found that the compositions of the invention are better at depositing anti-dandruff agents onto the scalp when the compositions also comprise a cationic polymer (such as cationic guar, for example), compared to corresponding compositions containing the anionic surfactant and the amphoteric or zwitterionic surfactant without the amphoalkanoate.

### pH

The pH of the compositions of the invention is preferably in the range of from 5 to 8, more preferably in the range of from 6 to 7 e.g., 6.5. The pH of the compositions of the invention can be adjusted using alkaline agents (such as sodium hydroxide, for example) or acidic agents (such as citric acid) as is well-known in the art.

It has surprisingly been found that the mildness of the compositions (that is to say the perception of the mildness of the composition on the hair and/or the scalp by the user of the composition) is particularly good in the pH ranges set out in the preceding paragraph.

### Product forms

The final product form of hair treatment compositions according to the invention may suitably be, for example, shampoos, conditioners, sprays, mousses, gels, waxes or lotions. Particularly preferred product forms are shampoos, post-wash conditioners (leave-in and rinse-off) and hair treatment products such as hair essences. Shampoos are a particularly preferred product form.

Preferably, the compositions are free of, or substantially free of, hair styling polymer.

Compositions in accordance with the invention are preferably formulated as compositions for the treatment of hair and subsequent rinsing.

A particularly preferred hair treatment composition in accordance with the invention is a shampoo composition. The total amount of surfactant in shampoo compositions of the invention (including any co-surfactant, and/or any emulsifier) is generally from 5 to 30%, preferably from 10 to 25%, more preferably from 15 to 20% by weight of the composition.

### Optional further surfactants

One or more further surfactants which are cosmetically acceptable and suitable for topical application to the hair may be present as an additional ingredient in shampoo compositions of the invention.

An example is a nonionic surfactant, which can be included in an amount ranging from 0 to 8, preferably from 2 to 5 wt%.

For example, representative nonionic surfactants that can be included in shampoo compositions of the invention include condensation products of aliphatic (C₈-C₁₈) primary or secondary linear or branched chain alcohols or phenols with alkylene oxides, usually ethylene oxide and generally having from 6 to 30 ethylene oxide groups.

Other representative nonionic surfactants include mono- or di-alkyl alkanolamides. Examples include coco mono- or diethanolamide and coco mono-isopropanolamide.

Further nonionic surfactants which can be included in shampoo compositions of the invention are the alkyl polyglycosides (APGs). Typically, the APG is one which comprises an alkyl group connected (optionally via a bridging group) to a block of one or more glycosyl groups. Preferred APGs are defined by the following formula:

RO-(G)ₙ

wherein R is a branched or straight chain alkyl group which may be saturated or unsaturated and G is a saccharide group.

R may represent a mean alkyl chain length of from about C₅ to about C₂₀. Preferably R represents a mean alkyl chain length of from about C₈ to about C₁₂. Most preferably the value of R lies between about 9.5 and about 10.5. G may be selected from C₅ or C₆ monosaccharide residues, and is preferably a glucoside. G may be selected from the group comprising glucose, xylose, lactose, fructose, mannose and derivatives thereof. Preferably G is glucose.

The degree of polymerisation, n, may have a value of from about 1 to about 10 or more. Preferably, the value of n lies in the range of from about 1.1 to about 2. Most preferably the value of n lies in the range of from about 1.3 to about 1.5.

Suitable alkyl polyglycosides for use in the invention are commercially available and include for example those materials identified as: Oramix NS10 ex Seppic; Plantaren 1200 and Plantaren 2000 ex Henkel.

Other sugar-derived nonionic surfactants which can be included in shampoo compositions of the invention include the C₁₀-C₁₈ N-alkyl (C₁-C₆) polyhydroxy fatty acid amides, such as the C₁₂-C₁₈N-methyl glucamides, as described for example in WO 92 06154 and US 5 194 639, and the N-alkoxy polyhydroxy fatty acid amides, such as C₁₀-C₁₈ N-(3-methoxypropyl) glucamide.

The shampoo composition can also optionally include one or more cationic co-surfactants included in an amount ranging from 0.01 to 10, more preferably from 0.05 to 5, most preferably from 0.05 to 2 wt%. Useful cationic surfactants are described hereinbelow in relation to conditioner compositions.

### Other optional components

Compositions of the invention may include one or more further optional components that can be used in hair treatment compositions. Examples of such optional components are given below.

### Cationic Polymer

A cationic polymer is a preferred ingredient in shampoo compositions of the invention, for enhancing conditioning performance of the shampoo.

The cationic polymer may be a homopolymer or be formed from two or more types of monomers. The molecular weight of the polymer will generally be between 5 000 and 10 000 000, typically at least 10 000 and preferably in the range 100 000 to about 2 000 000 g/mol. The polymers will have cationic nitrogen containing groups such as quaternary ammonium or protonated amino groups, or a mixture thereof.

The cationic nitrogen-containing group will generally be present as a substituent on a fraction of the total monomer units of the cationic polymer. Thus when the polymer is not a homopolymer it can contain spacer non-cationic monomer units. Such polymers are described in the CTFA Cosmetic Ingredient Directory, 3rd edition. The ratio of the cationic to non-cationic monomer units is selected to give a polymer having a cationic charge density in the required range.

Suitable cationic conditioning polymers include, for example, copolymers of vinyl monomers having cationic amine or quaternary ammonium functionalities with water soluble spacer monomers such as (meth)acrylamide, alkyl and dialkyl (meth)acrylamides, alkyl (meth)acrylate, vinyl caprolactone and vinyl pyrrolidine. The alkyl and dialkyl substituted monomers preferably have C1-C7 alkyl groups, more preferably C1-3 alkyl groups. Other suitable spacers include vinyl esters, vinyl alcohol, maleic anhydride, propylene glycol and ethylene glycol.

The cationic amines can be primary, secondary or tertiary amines, depending upon the particular species and the pH of the composition. In general secondary and tertiary amines, especially tertiary, are preferred.

Amine substituted vinyl monomers and amines can be polymerized in the amine form and then converted to ammonium by quaternization.

The cationic conditioning polymers can comprise mixtures of monomer units derived from amine- and/or quaternary ammonium-substituted monomer and/or compatible spacer monomers.

Suitable cationic conditioning polymers include, for example:
- copolymers of 1-vinyl-2-pyrrolidine and 1-vinyl-3-methyl-imidazolium salt (e.g. chloride salt), referred to in the industry by the Cosmetic, Toiletry, and Fragrance Association, (CTFA) as Polyquaternium-16. This material is commercially available from BASF Wyandotte Corp. (Parsippany, NJ, USA) under the LUVIQUAT tradename (e.g. LUVIQUAT FC 370);
- copolymers of 1-vinyl-2-pyrrolidine and dimethylaminoethyl methacrylate, referred to in the industry (CTFA) as Polyquaternium-11. This material is available commercially from Gaf Corporation (Wayne, NJ, USA) under the GAFQUAT tradename (e.g., GAFQUAT 755N);
- cationic diallyl quaternary ammonium-containing polymers including, for example, dimethyldiallyammonium chloride homopolymer and copolymers of acrylamide and dimethyldiallylammonium chloride, referred to in the industry (CTFA) as Polyquaternium 6 and Polyquaternium 7, respectively;
- mineral acid salts of amino-alkyl esters of homo-and co-polymers of unsaturated carboxylic acids having from 3 to 5 carbon atoms, (as described in U.S. Patent 4,009,256);
- cationic polyacrylamides(as described in WO95/22311).

Other cationic conditioning polymers that can be used include cationic polysaccharide polymers, such as cationic cellulose derivatives, cationic starch derivatives, and cationic guar gum derivatives. Suitably, such cationic polysaccharide polymers have a charge density in the range from 0.1 to 4 meq/g.

Cationic polysaccharide polymers suitable for use in compositions of the invention include those of the formula:

A-O-[R-N⁺(R¹)(R²)(R³)X⁻],

wherein: A is an anhydroglucose residual group, such as a starch or cellulose anhydroglucose residual. R is an alkylene, oxyalkylene, polyoxyalkylene, or hydroxyalkylene group, or combination thereof. R¹, R² and R³ independently represent alkyl, aryl, alkylaryl, arylalkyl, alkoxyalkyl, or alkoxyaryl groups, each group containing up to about 18 carbon atoms. The total number of carbon atoms for each cationic moiety (i.e., the sum of carbon atoms in R¹, R² and R³) is preferably about 20 or less, and X is an anionic counterion.

Cationic cellulose is available from Amerchol Corp. (Edison, NJ, USA) in their Polymer JR (trade mark) and LR (trade mark) series of polymers, as salts of hydroxyethyl cellulose reacted with trimethyl ammonium substituted epoxide, referred to in the industry (CTFA) as Polyquaternium 10. Another type of cationic cellulose includes the polymeric quaternary ammonium salts of hydroxyethyl cellulose reacted with lauryl dimethyl ammonium-substituted epoxide, referred to in the industry (CTFA) as Polyquaternium 24. These materials are available from Amerchol Corp. (Edison, NJ, USA) under the tradename Polymer LM-200.

Other suitable cationic polysaccharide polymers include quaternary nitrogen-containing cellulose ethers (e.g. as described in U.S. Patent 3,962,418), and copolymers of etherified cellulose and starch (e.g. as described in U.S. Patent 3,958,581).

A particularly suitable type of cationic polysaccharide polymer that can be used is a cationic guar gum derivative, such as guar hydroxypropyltrimonium chloride (commercially available from Rhone-Poulenc in their JAGUAR trademark series).

Examples are JAGUAR C13S, which has a low degree of substitution of the cationic groups and high viscosity. JAGUAR C15, having a moderate degree of substitution and a low viscosity, JAGUAR C17 (high degree of substitution, high viscosity), JAGUAR C16, which is a hydroxypropylated cationic guar derivative containing a low level of substituent groups as well as cationic quaternary ammonium groups, and JAGUAR 162 which is a high transparency, medium viscosity guar having a low degree of substitution.

Jaguar C17 is a particularly preferred deposition polymer for use in the compositions of the invention.

Preferably the cationic conditioning polymer is selected from cationic cellulose and cationic guar derivatives. Particularly preferred cationic polymers are JAGUAR C13S, JAGUAR C15, JAGUAR C17 and JAGUAR C16 and JAGUAR C162.

The cationic conditioning polymer will generally be present in compositions of the invention at levels of from 0.01 to 5, preferably from 0.05 to 1, more preferably from 0.08 to 0.5 wt%.

### Suspending Agents

In a preferred embodiment, the hair treatment composition, especially if it is a shampoo composition, further comprises from 0.1 to 5 wt% of a suspending agent for the coated particles. Suitable suspending agents are selected from polyacrylic acids, cross-linked polymers of acrylic acid, copolymers of acrylic acid with a hydrophobic monomer, copolymers of carboxylic acid-containing monomers and acrylic esters, cross-linked copolymers of acrylic acid and acrylate esters, heteropolysaccharide gums and crystalline long chain acyl derivatives. The long chain acyl derivative is desirably selected from ethylene glycol stearate, alkanolamides of fatty acids having from 16 to 22 carbon atoms and mixtures thereof. Ethylene glycol distearate and polyethylene glycol 3 distearate are preferred long chain acyl derivatives. Polyacrylic acid is available commercially as Carbopol 420, Carbopol 488 or Carbopol 493. Polymers of acrylic acid cross-linked with a polyfunctional agent may also be used, they are available commercially as Carbopol 910, Carbopol 934, Carbopol 940, Carbopol 941 and Carbopol 980. An example of a suitable copolymer of a carboxylic acid containing a monomer and acrylic acid esters is Carbopol 1342. All Carbopol (trade mark) materials are available from Goodrich.

Suitable cross-linked polymers of acrylic acid and acrylate esters are Pemulen TR1 or Pemulen TR2. A suitable heteropolysaccharide gum is xanthan gum, for example that available as Kelzan mu.

The suspending agent for the coated particles is preferably a polymeric suspending agent.

### Conditioning Agents

The compositions of this invention can also contain one or more conditioning agents selected from silicone conditioning agents and non-silicone oily conditioning agents.

When conditioning agent is present in the hair treatment compositions in droplet form, the droplets may be liquid, semi-solid or solid in nature, so long as they are substantially uniformly dispersed in the fully formulated product. Any droplets of oily conditioning agent are preferably present as either liquid or semi-solid droplets, more preferably as liquid droplets.

### Silicone Conditioning Agents

The compositions of the invention can contain, emulsified droplets of a silicone conditioning agent, for enhancing conditioning performance. The silicone is insoluble in the aqueous matrix of the composition and so is present in an emulsified form, with the silicone present as dispersed droplets.

Suitable silicones include polydiorganosiloxanes, in particular polydimethylsiloxanes which have the CTFA designation dimethicone. Also suitable for use compositions of the invention (particularly shampoos and conditioners) are polydimethyl siloxanes having hydroxyl end groups, which have the CTFA designation dimethiconol. Also suitable for use in compositions of the invention are silicone gums having a slight degree of cross-linking, as are described for example in WO 96/31188. These materials can impart body, volume and stylability to hair, as well as good wet and dry conditioning.

The viscosity of the emulsified silicone itself (not the emulsion or the final hair conditioning composition) is typically at least 10,000 cst. In general we have found that conditioning performance increases with increased viscosity. Accordingly, the viscosity of the silicone itself is preferably at least 60,000 cst, most preferably at least 500,000 cst, ideally at least 1,000,000 cst. Preferably the viscosity does not exceed 10⁹ cst for ease of formulation.

Emulsified silicones for use in the shampoo compositions of the invention will typically have an average silicone droplet size in the composition of less than 30, preferably less than 20, more preferably less than 10 µm. We have found that reducing the droplet size generally improves conditioning performance. Most preferably the average silicone droplet size of the emulsified silicone in the composition is less than 2 µm, ideally it ranges from 0.01 to 1 µm. Silicone emulsions having an average silicone droplet size of ≤ 0.15 µm are generally termed microemulsions.

Suitable silicone.emulsions for use in the invention are also commercially available in a pre-emulsified form.

Examples of suitable pre-formed emulsions include emulsions DC2-1766, DC2-1784, and microemulsions DC2-1865 and DC2-1870, all available from Dow Corning. These are all emulsions/microemulsions of dimethiconol. Cross-linked silicone gums are also available in a pre-emulsified form, which is advantageous for ease of formulation. A preferred example is the material available from Dow Corning as DC X2-1787, which is an emulsion of cross-linked dimethiconol gum. A further preferred example is the material available from Dow Corning as DC X2-1391, which is a microemulsion of cross-linked dimethiconol gum.

A further preferred class of silicones for inclusion in shampoos and conditioners of the invention are amino functional silicones. By "amino functional silicone" is meant a silicone containing at least one primary, secondary or tertiary amine group, or a quaternary ammonium group.

Examples of suitable amino functional silicones include:
(i) polysiloxanes having the CTFA designation "amodimethicone", and the general formula:

   HO-[Si(CH₃)₂-O-]ₓ-[Si(OH) (CH₂CH₂CH₂-NH-CH₂CH₂NH₂)-O-]_{y}-H

   in which x and y are numbers depending on the molecular weight of the polymer, generally such that the molecular weight is between about 5,000 and 500,000.
(ii) polysiloxanes having the general formula:

   R'ₐG₃₋ₐ-Si(OSiG₂)ₙ-(OSiG_{b}R'_{2-b})ₘ-O-SiG₃₋ₐ-R^{'}ₐ

   in which:
   G is selected from H, phenyl, OH or C₁₋₈ alkyl, e.g. methyl;
   a is 0 or an integer from 1 to 3, preferably 0;
   b is 0 or 1, preferably 1;
   m and n are numbers such that (m + n) can range from 1 to 2000, preferably from 50 to 150;
   m is a number from 1 to 2000, preferably from 1 to 10;
   n is a number from 0 to 1999, preferably from 49 to 149, and
   R is a monovalent radical of formula -C_{q}H_{2q}L in which q is a number from 2 to 8 and L is an aminofuctional group selected from the following:

      -NR"-CH₂-CH₂-N(R")₂

      -N(R")₂

      -N⁺(R")₃A⁻

      -N⁺H(R")₂A⁻

      -N⁺H₂(R")A⁻

      -N(R")-CH₂-CH₂-N⁺H₂(R")A⁻
   in which R is selected from H, phenyl, benzyl, or a saturated monovalent hydrocarbon radical, e.g. C₁₋₂₀ alkyl, and A is a halide ion, e.g. chloride or bromide.
   Suitable amino functional silicones corresponding to the above formula include those polysiloxanes termed "trimethylsilylamodimethicone" as depicted below, and which are sufficiently water insoluble so as to be useful in compositions of the invention:

   Si(CH₃)₃-O-[Si(CH₃)₂-O-]ₓ-[Si(CH₃)(R-NH-CH₂CH₂NH₂)-O-]_{y}-Si(CH₃)₃

   wherein x + y is a number from about 50 to about 500, and wherein R is an alkylene group having from 2 to 5 carbon atoms. Preferably, the number x + y is in the range of from about 100 to about 300.
(iii) quaternary silicone polymers having the general formula:

   {(R¹)(R²)(R³)N⁺CH₂CH(OH)CH₂O(CH₂)₃[Si(R⁴)(R⁵)-O-]ₙ-Si(R⁶)(R⁷)-(CH₂)₃-O-CH₂CH(OH)CH₂N⁺(R⁸)(R⁹)(R¹⁰)} (X⁻)₂
wherein R¹ and R¹⁰ may be the same or different and may be independently selected from H, saturated or unsaturated long or short chain alk(en)yl, branched chain alk(en)yl and C₅-C₈ cyclic ring systems;
R² thru' R⁹ may be the same or different and may be independently selected from H, straight or branched chain lower alk(en)yl, and C₅-C₈ cyclic ring systems; n is a number within the range of about 60 to about 120, preferably about 80, and
X is preferably acetate, but may instead be for example halide, organic carboxylate, organic sulphonate or the like. Suitable quaternary silicone polymers of this class are described in EP-A-0 530 974.

Amino functional silicones suitable for use in shampoos and conditioners of the invention will typically have a mole % amine functionality in the range of from about 0.1 to about 8.0 mole %, preferably from about 0.1 to about 5.0 mole %, most preferably from about 0.1 to about 2.0 mole %. In general the amine concentration should not exceed about 8.0 mole % since we have found that too high an amine concentration can be detrimental to total silicone deposition and therefore conditioning performance.

The viscosity of the amino functional silicone is not particularly critical and can suitably range from about 100 to about 500,000 cst.

Specific examples of amino functional silicones suitable for use in the invention are the aminosilicone oils DC2-8220, DC2-8166, DC2-8466, and DC2-8950-114 (all ex Dow Corning), and GE 1149-75, (ex General Electric Silicones).
Also suitable are emulsions of amino functional silicone oils with non ionic and/or cationic surfactant.

Suitably such pre-formed emulsions will have an average amino functional silicone droplet size in the shampoo composition of less than 30, preferably less than 20, more preferably less than 10 µm. Reducing the droplet size generally improves conditioning performance. Most preferably the average amino functional silicone droplet size in the composition is less than 2 µm ideally it ranges from 0.01 to 1 µm.

Pre-formed emulsions of amino functional silicone are also available from suppliers of silicone oils such as Dow Corning and General Electric. Specific examples include DC929 Cationic Emulsion, DC939 Cationic Emulsion, and the non-ionic emulsions DC2-7224, DC2-8467, DC2-8177 and DC2-8154 (all ex Dow Corning).

An example of a quaternary silicone polymer useful in the present invention is the material K3474, ex Goldschmidt.

For shampoo compositions according to the invention intended for the treatment of "mixed" hair (i.e. greasy roots and dry ends), it is particularly preferred to use a combination of amino functional and non-amino functional silicone in compositions of the invention, especially when these are in the form of shampoo compositions. In such a case, the weight ratio of amino functional silicone to non-amino functional silicone will typically range from 1:2 to 1:20, preferably 1:3 to 1:20, more preferably 1:3 to 1:8.
The total amount of silicone incorporated into compositions of the invention depends on the level of conditioning desired and the material used. A preferred amount is from 0.01 to 10 wt% although these limits are not absolute. The lower limit is determined by the minimum level to achieve conditioning and the upper limit by the maximum level to avoid making the hair and/or skin unacceptably greasy.

We have found that a total amount of silicone of from 0.3 to 5, preferably 0.5 to 3 wt% is a suitable level.

The viscosity of silicones and silicone emulsions can be measured by means of a glass capillary viscometer as set out further in Dow Corning Corporate Test Method CTM004, July 20 1970.

In compositions comprising silicone, it is preferred that a suspending agent for the silicone also be present. Suitable suspending agents are as described hereinabove.

### (ii) Non-silicone Oily Conditioning Components

Compositions according to the present invention may also comprise a dispersed, non-volatile, water-insoluble oily conditioning agent.

This component will be dispersed in the composition in the form of droplets, which form a separate, discontinuous phase from the aqueous, continuous phase of the composition. In other words, the oily conditioning agent will be present in the shampoo composition in the form of an oil-in-water emulsion.

By "insoluble" is meant that the material is not soluble in water (distilled or equivalent) at a concentration of 0.1% (w/w), at 25°C.

Suitably, the D_{3,2} average droplet size of the oily conditioning component is at least 0.4, preferably at least 0.8, and more preferably at least 1 µm. Additionally, the D_{3,2} average droplet size of the oily conditioning component is preferably no greater than 10, more preferably no greater 8, more preferably no greater than 5, yet more preferably no greater than 4, and most preferably no greater than 3.5 µm.

The oily conditioning agent may suitably be selected from oily or fatty materials, and mixtures thereof.

Oily or fatty materials are preferred conditioning agents in the shampoo compositions of the invention for adding shine to the hair and also enhancing dry combing and dry hair feel.

Preferred oily and fatty materials will generally have a viscosity of less than 5 Pa.s, more preferably less than 1 Pa.s, and most preferably less than 0.5 Pa.s, e.g. 0.1 Pa.s and under as measured at 25°C with a Brookfield Viscometer (e.g. Brookfield RV) using spindle 3 operating at 100 rpm.

Oily and fatty materials with higher viscosities may be used. For example, materials with viscosities as high as 65 Pa.s may be used. The viscosity of such materials (i.e. materials with viscosities of 5 Pa.s and greater) can be measured by means of a glass capillary viscometer as set out further in Dow Corning Corporate Test Method CTM004, July 20 1970.

Suitable oily or fatty materials are selected from hydrocarbon oils, fatty esters and mixtures thereof.

Hydrocarbon oils include cyclic hydrocarbons, straight chain aliphatic hydrocarbons (saturated or unsaturated), and branched chain aliphatic hydrocarbons (saturated or unsaturated). Straight chain hydrocarbon oils will preferably contain from about 12 to about 30 carbon atoms. Branched chain hydrocarbon oils can and typically may contain higher numbers of carbon atoms. Also suitable are polymeric hydrocarbons of alkenyl monomers, such as C₂-C₆ alkenyl monomers. These polymers can be straight or branched chain polymers. The straight chain polymers will typically be relatively short in length, having a total number of carbon atoms as described above for straight chain hydrocarbons in general. The branched chain polymers can have substantially higher chain length. The number average molecular weight of such materials can vary widely, but will typically be up to about 2000, preferably from about 200 to about 1000, more preferably from about 300 to about 600.

Specific examples of suitable hydrocarbon oils include paraffin oil, mineral oil, saturated and unsaturated dodecane, saturated and unsaturated tridecane, saturated and unsaturated tetradecane, saturated and unsaturated pentadecane, saturated and unsaturated hexadecane, and mixtures thereof. Branched-chain isomers of these compounds, as well as of higher chain length hydrocarbons, can also be used. Exemplary branched-chain isomers are highly branched saturated or unsaturated alkanes, such as the permethyl-substituted isomers, e.g., the permethyl-substituted isomers of hexadecane and eicosane, such as 2, 2, 4, 4, 6, 6, 8, 8-dimethyl-10-methylundecane and 2, 2, 4, 4, 6, 6-dimethyl-8-methylnonane, sold by Permethyl Corporation. A further example of a hydrocarbon polymer is polybutene, such as the copolymer of isobutylene and butene. A commercially available material of this type is L-14 polybutene from Amoco Chemical Co. (Chicago, Ill., U.S.A.).

Particularly preferred hydrocarbon oils are the various grades of mineral oils. Mineral oils are clear oily liquids obtained from petroleum oil, from which waxes have been removed, and the more volatile fractions removed by distillation. The fraction distilling between 250°C to 300°C is termed mineral oil, and it consists of a mixture of hydrocarbons ranging from C₁₆H₃₄ to C₂₁H₄₄. Suitable commercially available materials of this type include Sirius M85 and Sirius M125, all available from Silkolene.

Suitable fatty esters are characterised by having at least 10 carbon atoms, and include esters with hydrocarbyl chains derived from fatty acids or alcohols, e.g., monocarboxylic acid esters, polyhydric alcohol esters, and di- and tricarboxylic acid esters. The hydrocarbyl radicals of the fatty esters hereof can also include or have covalently bonded thereto other compatible functionalities, such as amides and alkoxy moieties, such as ethoxy or ether linkages.

Monocarboxylic acid esters include esters of alcohols and/or acids of the formula R'COOR in which R' and R independently denote alkyl or alkenyl radicals and the sum of carbon atoms in R' and R is at least 10, preferably at least 20.

Specific examples include, for example, alkyl and alkenyl esters of fatty acids having aliphatic chains with from about 10 to about 22 carbon atoms, and alkyl and/or alkenyl fatty alcohol carboxylic acid esters having an alkyl and/or alkenyl alcohol-derived aliphatic chain with about 10 to about 22 carbon atoms, benzoate esters of fatty alcohols having from about 12 to 20 carbon atoms.

The monocarboxylic acid ester need not necessarily contain at least one chain with at least 10 carbon atoms, so long as the total number of aliphatic chain carbon atoms is at least 10. Examples include isopropyl isostearate, hexyl laurate, isohexyl laurate, isohexyl palmitate, isopropyl palmitate, decyl oleate, isodecyl oleate, hexadecyl stearate, decyl stearate, isopropyl isostearate, dihexyldecyl adipate, lauryl lactate, myristyl lactate, cetyl lactate, oleyl stearate, oleyl oleate, oleyl myristate, lauryl acetate, cetyl propionate, and oleyl adipate. Di- and trialkyl and alkenyl esters of carboxylic acids can also be used. These include, for example, esters of C₄-C₈ dicarboxylic acids such as C₁-C₂₂ esters (preferably C₁-C₆) of succinic acid, glutaric acid, adipic acid, hexanoic acid, heptanoic acid, and octanoic acid. Examples include diisopropyl adipate, diisohexyl adipate, and diisopropyl sebacate. Other specific examples include isocetyl stearoyl stearate, and tristearyl citrate.

Polyhydric alcohol esters include alkylene glycol esters, for example ethylene glycol mono and di-fatty acid esters, diethylene glycol mono- and di-fatty acid esters, polyethylene glycol mono- and di-fatty acid esters, propylene glycol mono- and di-fatty acid esters, polypropylene glycol monooleate, polypropylene glycol monostearate, ethoxylated propylene glycol monostearate, polyglycerol poly-fatty acid esters, ethoxylated glyceryl monostearate, 1,3-butylene glycol monostearate, 1,3-butylene glycol distearate, polyoxyethylene polyol fatty acid ester, sorbitan fatty acid esters, polyoxyethylene sorbitan fatty acid esters and mono-, di-and triglycerides.

Particularly preferred fatty esters are mono-, di- and triglycerides, more specifically the mono-, di-, and triesters of glycerol and long chain carboxylic acids such as C₁-C₂₂ carboxylic acids. A variety of these types of materials can be obtained from vegetable and animal fats and oils, such as coconut oil, castor oil, safflower oil, sunflower oil, cottonseed oil, corn oil, olive oil, cod liver oil, almond oil, avocado oil, palm oil, sesame oil, peanut oil, lanolin and soybean oil. Synthetic oils include triolein and tristearin glyceryl dilaurate.

Specific examples of preferred materials include cocoa butter, palm stearin, sunflower oil, soyabean oil and coconut oil.

The oily or fatty material is suitably present at a level of from 0.05 to 10, preferably from 0.2 to 5, more preferably from about 0.5 to 3 wt%.

The compositions of this invention preferably contain no more than 3 wt% of a styling polymer, more preferably less than 1% of a styling polymer, preferably contain less than 0.1% by weight a styling polymer, and optimally are free of styling polymer.

In hair treatment compositions containing a conditioning agent, it is preferred that a cationic polymer also be present.

### Adjuvants

The compositions of the present invention may also contain adjuvants suitable for hair care. Generally such ingredients are included individually at a level of up to 2, preferably up to 1 wt% of the total composition.

Among suitable hair care adjuvants, are:
(i) natural hair root nutrients, such as amino acids and sugars. Examples of suitable amino acids include arginine, cysteine, glutamine, glutamic acid, isoleucine, leucine, methionine, serine and valine, and/or precursors and derivatives thereof. The amino acids may be added singly, in mixtures, or in the form of peptides, e.g. di- and tripeptides. The amino acids may also be added in the form of a protein hydrolysate, such as a keratin or collagen hydrolysate. Suitable sugars are glucose, dextrose and fructose. These may be added singly or in the form of, e.g. fruit extracts. A particularly preferred combination of natural hair root nutrients for inclusion in compositions of the invention is isoleucine and glucose. A particularly preferred amino acid nutrient is arginine.
(ii) hair fibre benefit agents. Examples are:
   - ceramides, for moisturising the fibre and maintaining cuticle integrity. Ceramides are available by extraction from natural sources, or as synthetic ceramides and pseudoceramides. A preferred ceramide is Ceramide II, ex Quest. Mixtures of ceramides may also be suitable, such as Ceramides LS, ex Laboratoires Serobiologiques.

### Minor ingredients

The compositions may also include conventional components such as colourants, fragrances and stabilisers.

The invention will now be further illustrated by the following, non-limiting Examples.

In the examples and throughout the specification, all percentages quoted are by weight based on total weight unless otherwise stated.

### EXAMPLES

### Example 1

The following is an example of a shampoo composition of the invention:

| **Component** | **Amount (wt%)** |
|---|---|
| Sodium lauryl ether sulphate (SLES) | 14 |
| Cocamidopropyl betaine (CAPB) | 2 |
| Sodium cocoamphoacetate (SCAA) | 2 |
| Climbazole | 1 |
| Silicone (DC1784)* | 2 |
| Jaguar C17** | 0.2 |
| NaOH | to pH 6.5 |
| preservative, fragrance | qv |
| Water | balance |

| | |
|---|---|
| *from Dow Corning **cationic guar from Rhodia | |

### Example 2

The deposition of climbazole from a composition of the invention containing Jaguar C17 was compared to deposition of climbazole from otherwise identical compositions containing different surfactant systems. The tests were carried out on skin in vitro. The results were as follows.

| Surfactant in composition | Relative deposition |
|---|---|
| 12 wt% SLES/ 2 wt% CAPB/ 2 wt % SCAA | 20.94 |
| 14 wt% SLES/ 2 wt% CAPB/ 2 wt % SCAA | 16.34 |
| 16 wt% SLES/ 2 wt% CAPB* | 11.33 |

| | |
|---|---|
| *comparative example | |

### Example 3

The mildness of the compositions of the invention was determined using in vitro corneosurfametry. The results were as follows.

| Surfactant in composition | Mildness |
|---|---|
| 14 wt% SLES/ 2 wt% CAPB/ 2 wt % SCAA | 39 |
| 16 wt% SLES/ 2 wt% CAPB | 27* |

| | |
|---|---|
| *comparative example | |

### Example 4

Sensory evaluation of the compositions of the invention was carried by a self perception questionnaire on a group of users. The compositions containing 14:2:2 SLES:CAPB:SCAA were found to be superior to compositions containing SLES and CAPB only, in terms of wet and dry attributes, including ease of comb and good alignment, and perceptible scalp benefits, such as no dandruff and scalp dryness.

## Claims

1. A hair treatment composition comprising:
(a) an anionic surfactant;
(b) an amphoteric or zwitterionic surfactant;
(c) an alkyl amphoalkanoate surfactant; and
(d) an anti-dandruff agent that is in solution in the composition, wherein the anti-dandruff agent is climbazole.

2. Composition as claimed in Claim 1, wherein the anionic surfactant is selected from sodium lauryl sulphate, sodium lauryl ether sulphate (n) EO, (where n ranges from 1 to 3), ammonium lauryl sulphate and ammonium lauryl ether sulphate(n)EO, (where n ranges from 1 to 3).

3. Composition as claimed in Claim 1 or Claim 2 wherein the anionic surfactant is present in an amount of from 10 to 20 % by weight of the composition.

4. Composition as claimed any one of the preceding claims wherein the amphoteric or zwitterionic surfactant is a betaine.

5. Composition as claimed any one of the preceding claims wherein the amphoteric or zwitterionic surfactant is cocamidopropylbetaine.

6. Composition as claimed in any one of the preceding claims wherein the amphoteric or zwitterionic surfactant is present in an amount of from 0.5 to 5% by weight of the composition.

7. Composition as claimed in any one of the preceding claims wherein the alkyl amphoalkanoate surfactant is a (C₆ to C₁₈ alkyl )ampho (C₁ to C₂ alkanoate).

8. Composition as claimed in any one of the preceding claims wherein the alkyl amphoalkanoate surfactant is sodium cocoamphoacetate or sodium lauryl cocoamphoacetate.

9. Composition as claimed in any one of the preceding, claims wherein the alkyl amphoalkanoate surfactant is present in the composition in an amount of from 0.5 to 5% by weight of the composition.

10. Composition as claimed in any one of the preceding claims, wherein the anti-dandruff agent is present in the composition in an amount of from 0.1 to 5% by weight.

11. Composition as claimed in any one of the preceding claims, wherein the composition comprises a cationic polymer in an amount of 0.01 to 5% by weight.

12. Composition as claimed in any one of the preceding claims which has a pH in the range of from 5 to 8.

13. Composition as claimed in any one of the preceding claims which has a pH in the range of from 6 to 7.

14. Composition as claimed in any one of the preceding claims which is a shampoo.

15. A method of treating hair which comprises applying to the hair a composition of any one of Claims 1 to 14.

## Patentansprüche

1. Haarbehandlungszusammensetzung, umfassend:
(a) ein anionisches Tensid;
(b) ein amphoteres oder zwitterionisches Tensid;
(c) ein Alkylamphoalkanoattensid; und
(d) ein Anti-Schuppenmittel, das in Lösung in der Zusammensetzung vorliegt, wobei das Anti-Schuppenmittel Climbazol ist.

2. Zusammensetzung nach Anspruch 1, worin das anionische Tensid ausgewählt ist aus Natriumlaurylsulfat, Natriumlaurylethersulfat (n)EO, (worin n im Bereich von 1 bis 3 liegt), Ammoniumlaurylsulfat und Ammoniumlaurylethersulfat (n)EO, (worin n im Bereich von 1 bis 3 liegt).

3. Zusammensetzung nach Anspruch 1 oder Anspruch 2, worin das anionische Tensid in einer Menge von 10 bis 20 Gewichtsprozent der Zusammensetzung vorliegt.

4. Zusammensetzung nach einem der vorangehenden Ansprüche, worin das amphotere oder zwitterionische Tensid ein Betain ist.

5. Zusammensetzung nach einem der vorangehenden Ansprüche, worin das amphotere oder zwitterionische Tensid Cocamidopropylbetain ist.

6. Zusammensetzung nach einem der vorangehenden Ansprüche, worin das amphotere oder zwitterionische Tensid in einer Menge von 0,5 bis 5 Gewichtsprozent der Zusammensetzung vorliegt.

7. Zusammensetzung nach einem der vorangehenden Ansprüche, worin das Alkylamphoalkanoattensid ein (C₆-C₁₈-Alkyl)ampho (C₁-C₂-alkanoat) ist.

8. Zusammensetzung nach einem der vorangehenden Ansprüche, worin das Alkylamphoalkanoattensid Natriumcocoamphoacetat oder Natriumlaurylcocoamphoacetat ist.

9. Zusammensetzung nach einem der vorangehenden Ansprüche, worin das Alkylamphoalkanoattensid in der Zusammensetzung in einer Menge von 0,5 bis 5 Gewichtsprozent der Zusammensetzung vorliegt.

10. Zusammensetzung nach einem der vorangehenden Ansprüche, worin das Anti-Schuppenmittel in der Zusammensetzung in einer Menge von 0,1 bis 5 Gewichtsprozent vorliegt.

11. Zusammensetzung nach einem der vorangehenden Ansprüche, worin die Zusammensetzung ein kationisches Polymer in einer Menge von 0,01 bis 5 Gewichtsprozent umfasst.

12. Zusammensetzung nach einem der vorangehenden Ansprüche, die einen pH-Wert in dem Bereich von 5 bis 8 aufweist.

13. Zusammensetzung nach einem der vorangehenden Ansprüche, die einen pH-Wert in dem Bereich von 6 bis 7 aufweist.

14. Zusammensetzung nach einem der vorangehenden Ansprüche, die ein Shampoo ist.

15. Verfahren zum Behandeln von Haar, das Auftragen einer Zusammensetzung nach einem der Ansprüche 1 bis 14 auf das Haar umfasst.

## Revendications

1. Composition de traitement capillaire comprenant :
(a) un tensioactif anionique ;
(b) un tensioactif amphotère ou zwitterionique ;
(c) un tensioactif à base d'amphoalcanoate d'alkyle ; et
(d) un agent anti-pelliculaire qui est en solution dans la composition, où l'agent anti-pelliculaire est le climbazole.

2. Composition selon la revendication 1, dans laquelle le tensioactif anionique est choisi parmi le lauryl sulfate de sodium, le lauryl éther sulfate (n) OE de sodium, (où n se situe dans la plage de 1 à 3), le lauryl sulfate d'ammonium et le lauryl éther sulfate (n) OE d'ammonium, (où n se situe dans la plage de 1 à 3).

3. Composition selon la revendication 1 ou la revendication 2, dans laquelle le tensioactif anionique est présent en une quantité de 10 à 20 % en poids de la composition.

4. Composition selon l'une quelconque des revendications précédentes, dans laquelle le tensioactif amphotère ou zwitterionique est une bétaïne.

5. Composition selon l'une quelconque des revendications précédentes, dans laquelle le tensioactif amphotère ou zwitterionique est une cocamidopropylbétaïne.

6. Composition selon l'une quelconque des revendications précédentes, dans laquelle le tensioactif amphotère ou zwitterionique est présent en une quantité de 0,5 à 5 % en poids de la composition.

7. Composition selon l'une quelconque des revendications précédentes, dans laquelle le tensioactif à base d'amphoalcanoate d'alkyle est un ampho(alcanoate en C₁ à C₂) d'alkyle en C₆ à C₁₈.

8. Composition selon l'une quelconque des revendications précédentes, dans laquelle le tensioactif à base d'amphoalcanoate d'alkyle est le cocoamphoacétate de sodium ou le lauryl cocoamphoacétate de sodium.

9. Composition selon l'une quelconque des revendications précédentes, dans laquelle le tensioactif à base d'amphoalcanoate d'alkyle est présent dans la composition en une quantité de 0,5 à 5 % en poids de la composition.

10. Composition selon l'une quelconque des revendications précédentes, dans laquelle l'agent anti-pelliculaire est présent dans la composition en une quantité de 0,1 à 5 % en poids.

11. Composition selon l'une quelconque des revendications précédentes, dans laquelle la composition comprend un polymère cationique en une quantité de 0,01 à 5 % en poids.

12. Composition selon l'une quelconque des revendications précédentes, qui possède un pH dans la plage de 5 à 8.

13. Composition selon l'une quelconque des revendications précédentes, qui possède un pH dans la plage de 6 à 7.

14. Composition selon l'une quelconque des revendications précédentes, qui est un shampooing.

15. Procédé de traitement capillaire qui comprend l'application sur les cheveux d'une composition selon l'une quelconque des revendications 1 à 14.
